# EUROPEAN PATENT APPLICATION

(11) **EP 3 372 691 A1**
(43) Date of publication of application: **12.09.2018**
(21) Application number: 17159353.6
(22) Date of filing: 06.03.2017
(51) Int. Cl.: C12Q 1/68

(54) **ARRANGEMENT FOR DETECTING AND AMPLIFYING A SIGNAL BASED ON A SINGLE TARGET MOLECULE AND METHOD FOR DETECTING AND AMPLIFYING A SIGNAL BASED ON A SINGLE TARGET MOLECULE**

(71) Applicant: Technische Universität Braunschweig, 38106 Braunschweig (DE)
(72) Inventor: Tinnefeld, Philip, 80999 München (DE); Vietz, Carolin, 38112 Braunschweig (DE); Lalkens, Birka, 38179 Schwülper (DE); Acuna, Guillermo, 38102 Braunschweig (DE)
(74) Representative: Gramm, Lins & Partner Patent- und Rechtsanwälte PartGmbB

(57) **Abstract**

In a first aspect, the present invention relates to an arrangement for detecting and amplifying a signal based on a single target molecule containing a target motive whereby the principle is based on plasmon resonance enhanced detection of said signal and the signal is quenched unless binding of this structure to the target motive of the single target molecule accordingly. In another embodiment, the present invention relates to a method for detecting and amplifying a signal based on a single target molecule containing a target motive with the arrangement according to the present invention. In addition, the use of said arrangement according to the present invention or the method according to the present invention for signal enhancement and/or single molecule analysis and/or its use in living cells is described.

## Description

In a first aspect, the present invention relates to an arrangement for detecting and amplifying a signal based on a single target molecule containing a target motive whereby the principle is based on plasmon resonance enhanced detection of said signal and the signal is quenched unless binding of the structure to the target motive of the single target molecule accordingly. In another embodiment, the present invention relates to a method for detecting and amplifying a signal based on a single target molecule containing a target motive with the arrangement according to the present invention. In addition, the use of said arrangement according to the present invention or the method according to the present invention for signal enhancement and/or single molecule analysis and/or its use in living cells is described.

### Prior Art

Single molecule techniques have a substantial influence on the developments in the nano-biotechnology field, such as superresolution microscopy or single molecule DNA sequencing. Also in the field of molecular diagnosis single molecules including biomarker or other marker molecules need to be detected specifically. In this connection, difficulties occur on the one hand in detecting specifically a single molecule even at low concentration and, on the other hand, in detecting the signal but not detecting surrounding molecules accordingly, thus, avoiding any background.

However, interrogating and using single molecules represents the ultimate goal of miniaturization. Reaching the goal of single molecule by assays is not merely a linear progression of common miniaturization and automation efforts for cast reduction by minimizing reagents but single molecules by assays will substantiate a new dimension of available information. In particular, this is because single molecule assays are the only technique to provide detailed information on the properties of molecules without averaging and their dynamics can be studied without the need for synchronization. This advantage is easiest visualized considering single molecule DNA sequencing. Today the technique of "next-next generation sequencing" becomes more and more commercial, that is, single molecule sequencing is in most approaches a single enzyme or a polymerase assay and has become the first industrial single molecule assay accordingly.

DNA molecules are determined as marker molecules being specific for diseases or as a biomarker in the diagnostic field. Often, optical methods are used including methods based on chromophores, like fluorophores, for detecting these markers accordingly. However, the systems based on single molecule detection all suffer from the fact that the signal of the chromophores, like fluorophores, used are at low concentration, thus, most of the detection systems are not equipped with suitable means for determining the signals at low levels. Amplification and enhancement of the signal is required allowing to obtain a distinct result.

As noted before, most of the methods described in the art allowing signal enhancement and signal amplification in molecular diagnosis are based on enzymatic amplification as it is the case for the PCR. In addition, signal enhancement in molecular assays are described e.g. in US 2013/0252825 A1 wherein the signal enhancement is possible by plasmon resonance. However, a problem of this technique is to position the marker molecule exactly in the field of plasmon resonance, thus, allowing electrical field enhancement of the signal accordingly. Recently Puchkova, A., et al, Nanoletters, 2015, 15, 8354-8359 described DNA origami in nano-antennas with over 5000 fold fluorescence enhancement and single molecule detection at 25 µM. That is, enhancement is possible by plasmon resonance.

Metal enhanced single molecular fluorescence using silver particle monomer and dimer due to coupling effect between metal particles is known in the prior art, e.g. described by Zhang, J et al, Nanoletters 2007, Vol. 7, No. 7, 2101 to 2107. Therein direct coupling of oligomers with silver particles is identified allowing enhancement of the fluorescence of a fluorescent dye located in between the coupled metal particles of the oligomer silver construct. The system described therein consists of nanoparticles coupled directly with single stranded oligonucleotides whereby said single stranded oligonucleotides allow hybridization to form double stranded oligonucleotides which are coupled with the fluorescence dye. The dimer formations of the nanoparticles by hybridization allow to generate the metal enhanced single molecule fluorescence on silver particle dimers.

Optical fluorescence sensors as well as methods for measuring the concentration of an analyte in a probe are described in the art whereby metallic nanoparticles are placed on a layer whereby the labelled analytes to be measured can bind to analyte bounding molecules placed between the nanoparticles, thus, said nanoparticles allow enhancement of the fluorescence.

Moreover, the use of metallic nanoparticles as plasmon may serve as auxiliary antenna for enhancing fluorescence of molecules, e.g. in the field of optical sensors. Coupling of electromagnetically radiated molecules, like chromophores, with a plasmon of the nanoparticles allow to influence intensity and dynamic of the interaction between molecules and light. Thus, it is possible to increase the emitting rate of fluorescent molecules.

On the other side, the main problem of using the reference approach is still to place the individual molecules on nano-apertures or in the hotspots of optical antennas, particularly, in the case of nano-apertures, the metal cladding used heavily deteriorates the fluorescence signal inside the nano-apertures making reliable methods or even quantifiable distance measurement e.g. by fluorescence resonance energy transfer (FRET) apparently impossible. Various factors influence fluorescence inside nano-apertures including the exponential itineration of light in the nano-aperture, local enhancement of the excitation intensity due to plasmonic effects, enhancement of the emission rates and change of the angular radiation pattern by the electromagnetic boundary condition and cavity effects as well as fluorescence quenching due to electronic and energy transfer to the metal cladding influence the brightness of the fluorophore. That is, the methods known in the art have various advantages and disadvantages, for example, most of the techniques involve laborious steps and expensive equipment as well as well trained staff. In addition, quite often laborious purification steps are required before allowing detection of the marker molecules accordingly. Hence, the known methods for single molecule detection need to be improved further.

In 2005, Rothemund et al, e.g. WO 2005/030978, identifies self-assembled DNA-structures, termed DNA-origami-technique. The main concept consists in providing a single DNA-strand, typically of more than 5000 base pairs, termed scaffold, folded with the help of several (typically more than 100) different staple strands. The self-assembly DNA structure is formed by mixing the single DNA strand together with staple strands, thus, forming the predetermined DNA structure. The so called DNA-origami-technique enables nano-scaffolding of the DNA, thus, creating arbitrary two and three dimensional shapes at the nano-scale. In addition, staple strands can be modified in a way that different structures, like fluorescent dyes or additional nucleic acid sequence can be added. These additional nucleic acid sequences act as specific binding sites protruding out of the nucleic acid nanostructures. Metallic nanoparticles can be functionalized with the complementary nucleic acid sequence, thus, upon mixing with the DNA nanostructures, these metallic nanoparticles can be bound to the nucleic acid nanostructures at specific locations. For example, Pall et al., J. Amp. Chem. Soc., 2011, 133, 17606-17609, describe the same accordingly.

In US 2013/0252825 A1, the technique of DNA-origami, combined with metallic nanoparticles is used to allow signal enhancement by plasmon resonance. In EP 2 940 150 A1 methods for detecting an analyte in a sample utilizing self-assembly of nucleic acids to yield predetermined nanostructures are described. The analyte is present if a nanostructure is formed.

### Brief description of the present invention

It is the aim of the present invention to overcome the limitations by known methods and to improve the detection of molecules with low concentration including single molecules, in particular, molecules including chromophores, like fluorophores, thus, a strong signal can be detected improving the detection of distinct signals at low concentrations. In particular, the aim of the present invention is to provide a method where locally the signal is enhanced, thus, a single signal, e.g. a signal based on a marker, like a label, can be detected locally at a low concentration, for example on single molecule level, by signal enhancement. The aim of the present invention is to provide methods and arrangements useful in detecting molecules at low concentration or single molecule studies by signal enhancement of said molecules to enhance said signals on single molecule level, allowing single molecule analysis at concentrations of the compounds to be measured at picomolar or nanomolar or smaller concentration. A first object is to provide an arrangement for amplifying a signal based on a single target molecule, in addition, methods for detecting and amplifying a signal based on a single target molecule is provided. In addition, kits or systems are disclosed including the arrangement for detecting and amplifying a signal based on a single target molecule based on a signal enhancement.

In a first aspect, the present invention relates to an arrangement for detecting and amplifying a signal based on a single target molecule containing a target motif comprising
a first structure having attached thereto a metallic nanostructure, like metallic nanoparticle(s), whereby said a metallic nanostructure, like metallic nanoparticle(s), is/are arranged to allow formation of an area of electrical field enhancement due to plasmon resonance after excitation with electromagnetic waves;
a second structure comprising a quencher moiety and a label moiety whereby said label moiety is quenched when the quencher moiety is present at this structure and no target molecule is bound to the second structure, characterized in that the second structure comprising a quencher moiety and a label moiety are positioned in the vicinity of the metallic nanostructure, like the metallic nanoparticle(s), like in between two metallic nanoparticles, whereby said metallic nanostructure, like the metallic nanoparticle(s), preferably at least two metallic nanoparticles, are arranged to allow formation of plasmon resonance and said second structure having a quencher moiety and a label moiety is positioned in said area of electrical field enhancement due to plasmon resonance, and wherein the second structure is a second structure having a motif being complementary to the target motif of the single target molecule to be determined.

In another aspect, the present invention relates to a method for detecting and amplifying a signal based on a single target molecule containing a target motif comprising the steps of:
- providing an arrangement according to the present invention;
- providing the target molecule containing the target motif;
- binding specifically the single target molecule to the second structure through binding of the target motif to the complementary target motif present in said second structure, thus, removing the quencher moiety from the vicinity of the label moiety,
- measuring the signal based on single target molecules by measuring the label excited by electromagnetic waves whereby said label is positioned in the area of electrical field enhancement due to plasmon resonance of a metallic nanostructure, like the metallic nanoparticle(s).

Moreover, the present invention relates to the use of any of the arrangements according to the present invention or a method according to the present invention for use in signal enhancement, in particular, enhancement of fluorescence and/or for use in single molecular analysis and/or for use in living cells.

Finally, the present invention relates to kits or systems for signal enhancement including the arrangement according to the present invention.

### Brief description of the drawings

Figure 1 is a scheme of the method according to the present invention, as well as the arrangement according to the present invention.

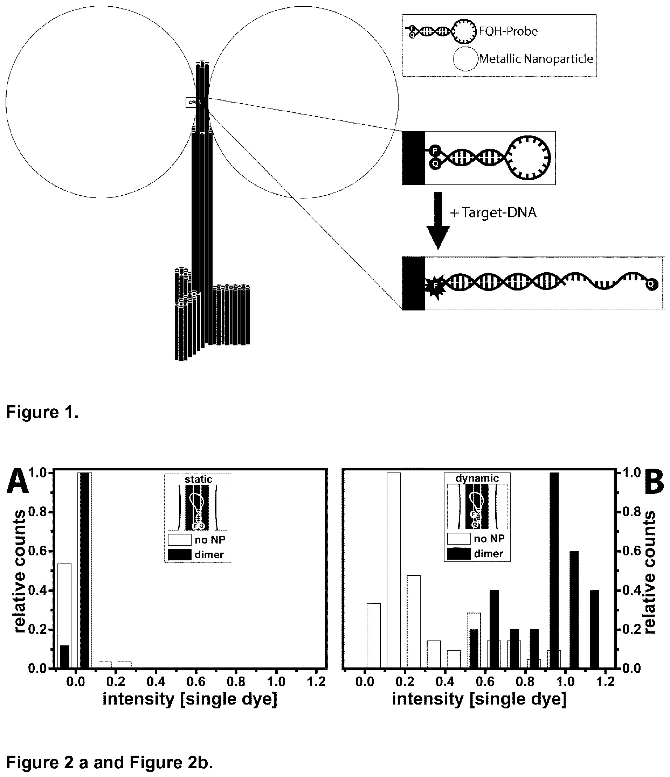

Figure 2a and 2b. Relative fluorescence intensity of the quenched fluorescence quenching hairpin probes (FQH). **A** relative intensities for static quenching for the dimer optical antenna (black) and without AgNP (white). The pair ATTO647N as dye and BBQ650 as quencher were exemplarily used. **B** relative intensities for dynamic quenching for the dimer optical antenna (black) and without AgNP (white), dimer refers to the presence of two nanoparticles forming in between a plasmon resonance field while no NP refers to the absence of nanoparticles, accordingly. The intensity is relative to a free single fluorophore (here, Atto647N).
Figure 2c. Total fluorescence enhancement of the FQH. Total enhancement for static (left) versus dynamic quenching (right) for the dimer antenna, monomer antenna and without AgNP, each.
Figure 2d shows a comparison of the fluorescence enhancement for FQH probes in the absence and presence of nanoparticles.
Figure 3 shows the overall enhancement disentangled in unquenching and plasmonic enhancement.
Figure 4 is a fraction of opened FQH probes after 24h incubation with 1 nM target DNA, 1 bp mismatched DNA, 2 bp mismatched DNA and 3 bp mismatched DNA with and without silver nanoparticles (80 nm). Nanoparticles do obviously not impair binding. In addition, it is demonstrated that the present invention allows to differentiate and to identify mismatching.

### Detailed description of the present invention

In a first aspect, the present invention relates to an arrangement for detecting and amplifying a signal based on a single target molecule containing a target motif comprising
a first structure having attached thereto a metallic nanostructure, like metallic nanoparticle(s), whereby said a metallic nanostructure, like metallic nanoparticle(s), is/are arranged to allow formation of an area of electrical field enhancement due to plasmon resonance after excitation with electromagnetic waves;
a second structure comprising a quencher moiety and a label moiety in close vicinity whereby said label moiety is quenched when the quencher moiety is present at this structure and no target molecule is bound to the second structure, characterized in that the second structure comprising a quencher moiety and a label moiety are positioned in the vicinity of the metallic nanostructure, like the metallic nanoparticle(s), like in between two metallic nanoparticles, whereby said metallic nanostructure, like the metallic nanoparticle(s) preferably at least two metallic nanoparticles, are arranged to allow formation of plasmon resonance and said second structure having a quencher moiety and a label moiety is positioned in said area of electrical field enhancement due to plasmon resonance, and wherein the second structure is a second structure having a motif being complementary to the target motif of the single target molecule to be determined.

That is, the present inventors provide an arrangement for detecting and amplifying a signal based on a single target molecule wherein the label/quencher system is combined with the plasmon resonance system. Surprisingly, the quenched label is not enhanced by plasmon resonance, thus, remain undetectable while the non-quenched label moiety can be enhanced due to plasmon resonance after excitation with electromagnetic waves. This is surprising since it was expected that not only the label signal in the non-quenched condition is enhanced by plasmon resonance but also the quenched signal of the active quencher/label system. Hence, using this combined system of quenching the label and, after unquenching, enhancement of said label by plasmon resonance allow the detection and amplification of a signal based on a single target even at low concentrations. That is, the dynamic range of the signal is increased from quenched to significantly enhanced. To put it another way, the contrast is higher due to the larger difference between the quenched signal and the non-quenched, enhanced signal.

In this connection the terms "enhancement" or "enhancing" and "amplification" or "amplified" are used interchangeably herein unless otherwise defined.

The term "arrangement" as used herein refers to a set or kit containing the components identified. That is, the arrangement contains at least the first structure, the second structure as defined. Typically, the arrangement of said structure are on an array.

The term "array" as used herein, refers to a substrate containing e.g. multiple of nucleic acid molecules. In one embodiment of the present invention, the array is a nucleic acid based origami molecule or a nucleic acid based chip. For example, the array is a DNA-origami containing various single stranded DNA extensions being arranged adjacent to one another.

"A quencher moiety and a label moiety in close vicinity" refers to an arrangement of the two moieties for static quenching where quencher moiety and label moiety are able to touch each other. Static quenching solely occurs with no distance between them. Within a distance of 9 nt static quenching partly occurs together with another quenching mechanism, namely dynamic quenching which again solely occurs from a distance of 10 nt and up, see e.g. Marras S. A. E., et al. Nucleic Acid Res. 2002, 30(21),e122. That is, the distance of quencher moiety and label moiety is below 10 nt (nucleotides), like below 9 nt, below 8 nt, below 7 nt, below 6 nt, below 5 nt, below 4 nt, below 3 nt, below 2 nt, below 1 nt. The term "static quenching" is also known as "contact mediated quenching".

In the label quencher system as used herein, the label is quenched when in the presence of the quencher, the fluorescence intensity is below 10% of the fluorescence intensity in the absence of the quencher, e.g. is below 9 %, 8 %, 7%, 6 %, 5 %, 4 %, 3 %, 2 %, or 1 %.

By combining the key elements from several state of the art nanotechnologies, here nanophotonics, nucleic acid nanotechnologies and for detecting purposes, here single-molecules-spectroscopy, it is possible to provide arrangements and methods for detecting and amplifying a signal based on a single target molecule accordingly. These arrangements are particularly suitable for single molecule assays and signal enhancement and molecular, preferably, single molecule assays based on specifically positioning the quencher/label moiety in the area of electrical field enhancement due to plasmon resonance after excitation with electromagnetic waves. Typically, this label moiety together with a quencher moiety is in the vicinity of a metallic nanostructure, like the metallic particles, preferably, in between the at least two metallic nanoparticles whereby said metallic nanostructure, like nanoparticles, are arranged to allow formation of plasmon resonance.

The first structure present in the arrangement according to the present invention is in an embodiment a structure having attached thereto metallic nanoparticle. Said metallic nanoparticle may be arranged as one nanoparticle or with at least two metallic nanoparticles. Said metallic nanoparticles are arranged in a way to allow formation of an area of electrical field enhancement due to plasmon resonance.

Further, the metallic nanostructure arranged to allow formation of an area of electric field enhancement due to plasmon resonance after excitation with electromagnetic waves may be metallic nanoparticles, metallic nanorods, metallic cubes, metallic prisms, metallic pyramids, metallic wires, metallic nanostars, metallic nanoshells, metallic films, metallic nanostructures created by lithography including periodic plasmonic structures like metallic nanoarrays or pillar arrays, metallic nanostructures fabricated by nanoimprinting and metallic nanostructures made by other self-assembly processes as well as other structures that lead to an electric field enhancement.

The term "electrical field enhancement due to plasmon resonance" refers to the increase of the electric field in the vicinity of a metallic nanostructure, like a metallic nanoparticle, due to the polarization of the metallic nanostructure, like the nanoparticle, induced by the incoming oscillating electric field (e.g. light).

In particular, the use of optical antenna for signal enhancement based on electrical field enhancement due to plasmon resonance is well known in the art. That is, after exciting the compound in the vicinity of the metallic nanostructure, like metallic nanoparticle, or in between the at least two metallic nanoparticles, those metallic nanostructure, like the nanoparticle(s) form a local electric field which interacts with the compound. The signal is enhanced by the local electric field formed in the vicinity of the metallic nanostructure, like the metallic particle or in between the metallic particles allowing enhancement of the eradiated signals of the compounds accordingly.

Similarly, the radiative and non-radiative rates of the chromophore, like a fluorophore are altered, which can lead to a strong increase of the chromophore, like fluorescence quantum yield. That means, a dye that is usually a weak fluorophore or chromophore becomes a good chromophore, like fluorophore, while a good chromophore, like fluorophore, usually stays a good chromophore, like fluorophore, and only benefits from the excitation enhancement.

Not be bound to theory, it is submitted that due to plasmon resonance, the local electric field on that metallic nanostructure, like metallic particles, is formed. This electrical field allows enhancement of the signal eradiated from the compound in the hotspot of said particle(s). In this connection, the term "hotspot" is used herein to identify the area of electrical field enhancement accordingly.

Surprisingly, it has been recognized by the present inventors that the construct of quencher and label moiety whereby the label moiety is quenched when the quencher moiety is present in close vicinity, does not result in a detectable enhancement by plasmon resonance which can be expected but there is no detectable signal even in the electrical field due to plasmon resonance.

In case of removing the quencher from the label or the quencher is spaced apart from the label, e.g. 10 nt or more, the signal of the label is significantly enhanced by the electric field due to plasmon resonance. Hence, it is possible to amplify the signal based on a single target molecule dramatically.

In a preferred embodiment, the compound able to irradiate electronic waves due to plasmon resonance is a chromophore in particular, a fluorophore. It is preferred that the size of the metallic nanoparticle is below the exciting wave length to allow plasmon rensonance. Preferably, the metallic nanoparticles have a mean size of from 10 nm to 500 nm, preferably of from 10 nm to 200 nm.

Further, it is preferred that the metallic nanoparticles are in form of rods or spheres. They can also be in form of triangles or other types of design including films creating plasmonic structures or structures created by lithography representing said metallic nanostructures. However, it is also possible that the metallic nanoparticles may be in pyramidal form or may have an irregularly shape depending on the type of the signal enhancement and the method for detection of the signal.

Further, it is preferred that the metallic nanostructure, like metallic nanoparticles, are selected from plasmonic materials selected from silver, aluminum, gold, copper, palladium, and/or platinum. The skilled person is well aware of suitable metallic materials and suitable metallic nanoparticles allowing the formation of these in kind of optical antenna.

As used herein, the term "in the vicinity" with respect to the label (quencher) and the metallic nanostructure refers to distance or area between the compound and the metallic particle being in the nanometer range, e.g. in the range of from 1 to 200 nm, like 2 to 200 or 2 to 25 nm. The skilled person is well aware of the size of the electrical field formed around the metallic nanostructure, like the metallic nanoparticles due to plasmon resonance. Said distance or area is also called the near-filed area.

In case of at least two nanoparticles it is preferred that the spacing of said at least two nanoparticles is from 2 nm to 50 nm, thus, signal enhancement due to electrical field enhancement based on plasmon resonance is achieved.

It is particularly preferred that the orientation of the particles is adapted to the polarization of the exciting electromagnetic waves. By positioning the metallic nanostructure, like the metallic nanoparticles or by arranging the same at the first structure whereby this first structure is a nucleic acid based structure, it is possible to bind the second structure at a predetermined size in the vicinity of the metallic nanostructure, like the metallic nanoparticles, thus, positioning the quencher/label system in the electrical field where enhancement takes place due to plasmon resonance after being excited with electromagnetic waves accordingly.

The metallic nanostructure, like the metallic nanoparticles, may be bound at predetermined positions to nucleic acid molecules in the arrangement that is, a nucleic acid sequence having attached said metallic nanostructure, like the metallic nanoparticles. The nucleic acid sequences are able to hybridize to specific segments present on the base structure, like a carrier or an array. Alternatively or in addition, the metallic nanostructure, like the metallic nanoparticles may be bound to nucleic acid molecules, in particular, DNA molecules covalently, e.g. by linker groups. The metallic nanostructure, like the metallic nanoparticles are arranged with the first structure to allow formation of an area of electrical field enhancement due to plasmon resonance after exciting with electromagnetic waves as described above.

The first structure may be attached to a surface by covalent or non-covalent linkage. As noted before, the surface may be a DNA origami structure or may be any other kind of surface including arrays.

For example, the DNA of the first structure or of the DNA origami representing the array included biotin molecules which may be attached to the surface with the help of its counterpart, like avidin or streptavidin. Of course, other possibilities of attaching these structures to the surface can be envisaged. By attaching the predetermined structure on the surface allows to arrange the metallic nanostructure, like the nanoparticles, with respect to polymerization of the exciting light. The surface including the array is typically a surface of a layer allowing to pass the electromagnetic waves.

Suitable layers include glass, ITO glass, fused silica etc.

The label which is a compound able to irradiate electromagnetic waves is positioned in form of the label/quencher system in the vicinity of the metallic nanostructure, like metallic nanoparticles, like in between two metallic nanoparticles positioned in a way to allow formation of an electrical field enhancement by plasmon resonance. Thus, the quencher/label system is positioned in the "hotspot", i.e. in the area of the electrical field enhancement due to the plasmon resonance formed by a metallic nanostructure, like the metallic nanoparticle(s), when excited with electromagnetic waves. The compound is positioned at a sufficient distance from a metallic nanostructure, like the metallic nanoparticle(s), to enhance the irradiated signal when excited by an irradiating source.

The label/quencher system is a system known to the skilled person in the art. Typically, the label is a chromophore, like a fluorophore. The skilled person is well aware of suitable chromophores including fluorophores. Typically, the fluorescent dyes are used in fluorescence based techniques including the label/quencher system of ATTO647N and BBQ650. Other systems and other quenchers are known to the skilled person.

In an embodiment, the label quencher system is the ATTO647N and BBQ650 system.

The target motif is typically a nucleic acid containing motif. However, the target motif may also be a part of a ligand/receptor complex or other known interacting complexes whereby one part represents the target motif and the other part represents the complementary motif.

The term "nucleic acid molecules" includes all the structures containing parts or consisting of other nucleic acid structures, like RNA, PNA etc. The skilled person is well aware of suitable nucleic acid molecules which can be employed in the present invention.

In an embodiment, the first structure and/or second structure comprises nucleic acid sequences, in particular, DNA and/or RNA sequences.

Further, the arrangement according to the present invention in one embodiment has a target motif being a target sequence of nucleic acids.

In another embodiment, the arrangement according to the present invention is an arrangement wherein the second structure is a molecule having single strand nucleic acid sequence binding specifically to a target sequence of nucleic acids whereby said single strand nucleic acid sequence has a quencher moiety and a label moiety and said label is quenched when the quencher moiety is present in close vicinity at this structure and the target sequence is absent.

That is, the second structure is for example a structure containing hairpin structures also known as molecular beacons where the system in the molecular beacon is detached due to binding of the complementary sequence to the target motif present in the second structure of the single target molecule containing the target motif to be detected. Another example is a strand displacement probe, a duplex scorpion primer or amplifluor primer, also described generally as fluorescent nucleic acid hybridization probes.

After binding of the target sequence with its target motif to the sequence complementary to said target motif, the quencher/label system is detached or separated, thus, the quencher moiety does no longer quench the label, like the chromophore and, in particular, the fluorophore.

Only after binding of the target motif present in the single target molecule, a signal can be detected. By combining this technique of quencher/label system with the plasmon resonance technique, it is possible to enhance said signal by plasmon resonance after excitation with electromagnetic waves.

In another embodiment, the arrangement is an arrangement wherein the first structure is a nucleic acid sequence having attached thereto a metallic nanostructure, like metallic nanoparticle(s), optionally, whereby said nucleic acid sequence is having attached thereto a metallic nanostructure, like the metallic nanoparticle(s), are positioned at predetermined segments of a two or three dimensional structure of predetermined shape whereby said two or three dimensional structure is formed of nucleic acids, in particular, DNA molecules. In a preferred embodiment, these two or three dimensional structures of predetermined shape are DNA origami structures or similar structures known in the art as DNA nanostructures including gridiron structures, tiles, single-stranded tiles (sst), bricks, and 3D meshes.

In a further embodiment, a metallic nanostructure, like the metallic nanoparticles, being bound to the two or three dimensional structure of predetermined shape whereby said structure is formed of nucleic acid molecules, in particular, DNA molecules, a metallic nanostructure, like the metallic nanoparticles, are arranged to allow formation of an area of electrical field enhancement due to plasmon resonance after excitation with electromagnetic waves.

In another embodiment, the two or three dimensional structure of predetermined shape in the arrangement according to the present invention is an array, preferably, comprising a multiple of nucleic acid molecules, in particular, a nucleic acid based origami structure.

As noted before, the second structure may be a structure having a hairpin structure when no target sequence binds to the second structure, thus, the label and the quencher are present at the neck of the hairpin structure in close vicinity whereby the label is quenched with the quencher moiety.

Further, the second structure contains a motif being complementary to the target motif of the single target molecule to be determined. Typically, in case of nucleic acid sequences composing this motif, the complementarity is a complementarity being at least 90 %, preferably, 95 % like 96, 97, 98, 99 or 100 % complementarity to the target motif of the single target molecule.

Typically, the target motif in case of nucleic acid sequences is in the range of from 12 to 100, for example, the targeting motif has a size of 16 to 60 nucleic acid length.

The detachment or separation of the quencher moiety to the label moiety is given only when the target molecule hybridizes or binds to the second structure through the target motive, thus, the quencher does no longer quench the label accordingly. Said dequenching may be achieved by cleaving of the quencher or simply by eliminating the hairpin structure. Alternatively, the detachment of the system may be achieved by strand displacement.

The skilled person is well aware of suitable molecules.

As noted, the array may be a DNA origami. A DNA origami is a nanostructure formed from a long scaffold strand and many small, so called staple strands. Alternatively, the array can be composed of alternative DNA nanostructures such as single stranded tiles or gridiron structures or related, see e.g. Bryan Why, et al in doi:10.1038/nature075 Nature 2012. Of course, other nucleic acid based structures are possible as well as substitutes of DNA, like RNA, PNA, LNA etc.

In a further embodiment, the present invention relates to a method for detecting and amplifying a signal based on a single target molecule containing a target motif comprising the steps of:
- providing an arrangement according to the present invention;
- providing the target molecule containing the target motif;
- binding specifically of a single target molecule to the second structure through binding of the target motif to the complementary target motif present in said second structure, thus, removing the quencher moiety from the vicinity of the label moiety,
- measuring the signal based on single target molecules by measuring the label excited by electromagnetic waves whereby said label is positioned in the area of electrical field enhancement due to plasmon resonance of a metallic nanostructure, like the metallic nanoparticle(s).

The method according to the present invention is based on the combination of the enhancement of the signal due to electrical field enhancement by plasmon resonance whereby the signal is determined only after detachment of the quencher moiety from the label moiety. Detachment of the quencher moiety from the label moiety occurs only in case of binding of the single target molecule containing a target motif to the second structure, either directly or indirectly whereby said second structure contains a complementary target motif.

Plasmon resonance is induced typically with a suitable source for exciting with electronic waves. The skilled person is well aware of suitable sources for exciting with electromagnetic waves, like laser or other sources. The laser may emit electromagnetic waves with a specific wavelength or with a broad range of electromagnetic wave lengths. Typically, the electromagnetic waves according to the present invention is light, typically, visible light.

The signal based on the single target molecules by measuring the label excited by electromagnetic waves whereby said label is positioned in the area of electrical field enhancement due to plasmon resonance of a metallic nanostructure, like the metallic nanoparticle(s), is conducted by suitable systems. Said systems include spectroscopic systems including microscopy, dark-field microscopy, scattering microscopy but also Raman-spectroscopy like surface enhanced Raman-spectroscopy, surface enhanced resonance Raman-spectroscopy and fluorescence correlation spectroscopy.

The method according to the present invention allows to perform single molecular studies at elevated concentrations of the compounds to be tested as well as elevated concentrations of the label structure in so far that it has been recognized for the first time that a structure of label/quencher system does not result in an enhancement of any signals in an electrical field by plasmon resonance as expected in the art. In contrast, after detaching the quencher moiety from the label moiety, a significant signal enhancement occurs.

The signal to be detected is increased only in the presence of the target molecule. In addition, it is possible to increase the time resolution. Further, additional fluorophores become amenable to single molecular analysis since fluorescence quantum yields can be enhanced.

The method according to the present invention as well as the arrangement according to the present invention allows to create a platform for a single molecule lap on a chip allowing parallel investigations of different reactions, binding assays and e.g. drug screening assays in confined volumes. In an embodiment of the present invention, the label is a fluorophore and excitation is by laser light, thus, measurement is of fluorescence, e.g. by microscope or other spectroscopic systems.

Hence, the drawbacks of single molecule and single target analysis described in the art can be overcome.

In another embodiment, the present invention relates to the use of the arrangement according to the present invention or the method according to the present invention for at least one of signal enhancement, single molecule analysis and/or for use in living cells. The use in living cells includes *in vitro* use accordingly. In an embodiment, signal enhancement relates to enhancement of fluorescence. Typically, the fluorescent enhancement according to the present invention in the arrangement or the method according to the present invention is at least of a factor of 10 times, e.g. at least 20 times, like at least of a factor of 50 times, in particular, it is possible to have a signal enhancement factor of 500 times, like 1000 times, e.g. 5000 times without any background signal in case of the presence of the quencher/label complex in the electrical field due to plasmon resonance.

The method according to the present invention may also be used to determine mismatches including single nucleotide polymorphisms in a nucleic acid molecule obtained from a subject. That is, under appropriate conditions, namely under appropriate hybridization conditions, the complementary sequence of the target motif allows to differentiate between fully complementary sequences or sequences having one or two or three or more mismatches in the targeting motif of the target molecule. Thus, this system is particularly suitable for identifying any SNPs or other types of polymorphisms accordingly. That is, depending on the number of mismatches and depending on the hybridization conditions, a signal may be detectable or not. The signal enhancement depends on the number of mismatches between the target sequence and the complementary sequence present on the second structure as well as the hybridization conditions applied.

The method according to the present invention is useful for various purposes and approaches including determining analytes at low concentrations of picomolar or nanomolar ranges, e.g. single molecule level in a sample, for diagnostics based on biomarkers as well as in the field of chip technology. In addition, the method can be used to determine polymorphisms and for signal amplification in general.

In a further aspect, the present invention relates to a kit or system for signal enhancement of a label or marker including chromophores, in particular fluorophores, comprising the arrangement according to the present invention or comprising at least the first structure as defined herein and the second structure as defined herein, optionally, containing in addition devices for exciting electromagnetic waves as well as for measuring the excited electromagnetic waves, in particular, a fluorescence.

The present invention will be described further by way of examples without limiting the present application thereto.

Figure 1 is a sketch of the DNA origami nanoantenna with two 80 nm silver nanoparticles. The fluorescence quenching hairpin (FQH) probe is placed in the hotspot. Unquenching occurs upon binding of the target DNA shown in the embedded figures. Of note, it is possible that not only one arrangement is present at the origami nanoantenna but it is possible to place more than one hairpin targeting molecule, or second structure in general, on one DNA origami. That is, two or more hairpin targeting molecules or second structures in general, may be present within the DNA origami nanoantenna. This means that it is possible to have at least two identical or at least two different second structures within the area of electrical field enhancement due to plasmon resonance after excitation with electromagnetic waves.

That is, different hairpins in the hotspot may have different labels resulting in different colours allowing detection of more than one target sequence. Further in an embodiment of the present invention, more than one second molecules are present having the same label allowing further enhancement of the signal.

### Example 1

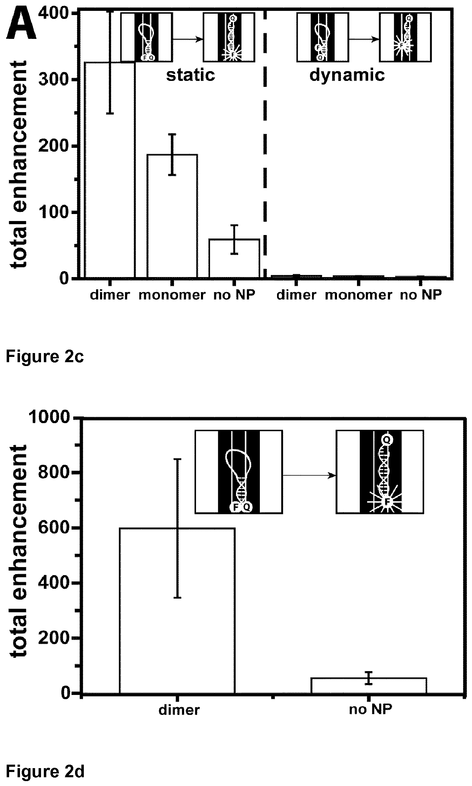

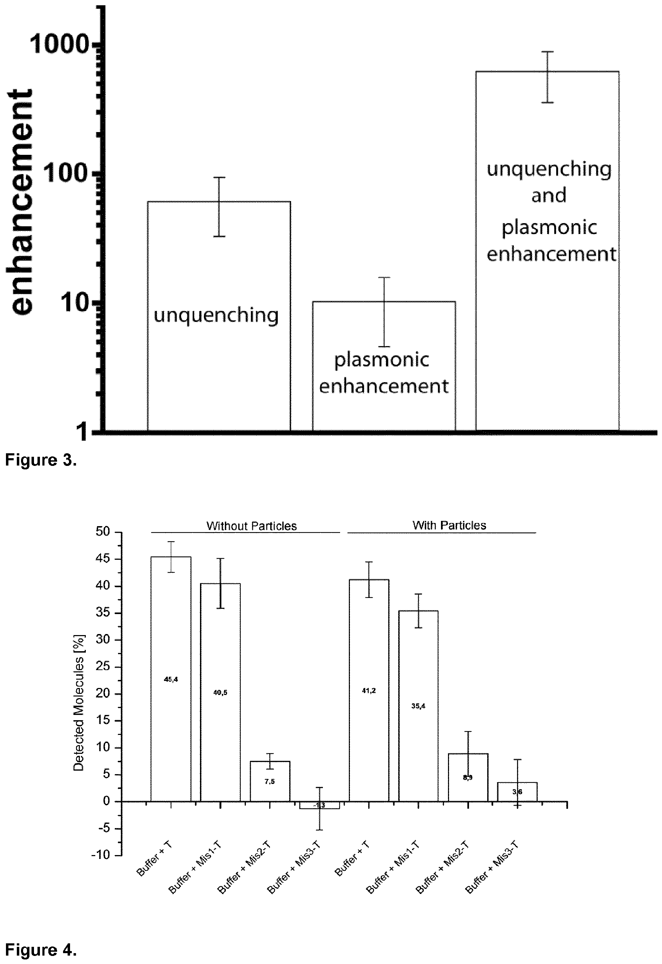

Comparison of the fluorescence enhancement in the absence or presence of nanoparticles. According to figure 1 a fluorescence quenching hairpin (15 nt stem, 15 nt loop, BBQ650 as quencher moiety and Atto647N as label moiety) is placed in the hotspot of the optical nanoantenna and fluorescence intensities are recorded before and after target DNA (30 nt) addition, thus for the quenched and unquenched or opened conformation of the fluorescence quenching hairpin. The experiments were performed for a fluorescence quenching hairpin with a fluorophore and quencher touching each other (static quenching) and a fluorophore and quencher 10 nt away from each other (dynamic quenching). In both cases the same target DNA was used and incubated for 20 h at room temperature. Confocal single molecule measurements were performed to obtain fluorescence intensities. Measurements were repeated the same way, only without nanoparticles. So, no plasmonic fluorescence enhancement occurs. Fluorescence intensities of the quenched conformation for dimer optical antenna and without nanoparticles were normalized and depicted in figure 2a and figure 2b. In presence of silver nanoparticles the fluorescence intensities increase for the dynamic quenching hairpin while it remains the same for the static quenching hairpin. This suggests a modularity of plasmonic enhancement and fluorescence quenching only for static quenching. To further show this modularity fluorescence intensities of the quenched and the opened conformation of the same nanostructure were compared to result in total enhancement (figure 2c, 2d). While the total enhancement for static quenching is up to 600-fold, there is almost no enhancement (up to 4) for dynamic quenching proving that there is only a modularity for plasmonic enhancement and static quenching and not for plasmonic enhancement and dynamic quenching. The modularity is further demonstrated in figure 3 by entangling the total enhancement in enhancement due to unquenching and plasmonic enhancement.

### Example 2

Detection of mismatches. A fluorescence quenching hairpin (12 nt stem, 12 nt loop) was positioned in the hotspot of the nanoantenna (see figure 1). Target DNA sequences (23 nt) with no, one, two or three mismatches were added to the reaction chambers each and incubated for 18 h at -4 °C. Confocal single molecule experiments were performed to detect the number of opened fluorescence quenching hairpins compared to the total number of nanostructures. Measurements were repeated without nanoparticles to control whether nanoparticles influence the fluorescence quenching hairpin opening. Results show that the fluorescence quenching hairpin is sensitive towards single nucleotide mismatches and the presence of nanoparticles does not influence the opening of the fluorescence quenching hairpin.

## Claims

1. An arrangement for detecting and amplifying a signal based on a single target molecule containing a target motif comprising
a first structure having attached thereto a plasmonic nanostructure, like metallic nanoparticle(s), whereby said plasmonic nanostructure, like metallic nanoparticle(s), is/are arranged to allow formation of an area of electrical field enhancement due to plasmon resonance after excitation with electromagnetic waves;
a second structure comprising a quencher moiety and a label moiety whereby said label moiety is quenched when the quencher moiety is present at this structure and no target molecule is bound to the second structure, **characterized in that** the second structure comprising a quencher moiety and a label moiety are positioned in the vicinity of the plasmonic nanostructure, like metallic nanoparticle(s), like in between two metallic nanoparticles, whereby said a plasmonic nanostructure, like metallic nanoparticle(s), preferably at least two metallic nanoparticles, are arranged to allow formation of plasmon resonance and said second structure having a quencher moiety and a label moiety is positioned in said area of electrical field enhancement due to plasmon resonance, and wherein the second structure is a second structure having a motif being complementary to the target motif of the single target molecule to be determined.

2. The arrangement according to claim 1 wherein the first structure and/or second structure comprises nucleic acid sequences, in particular, DNA and/or RNA sequences.

3. The arrangement according to claim 1 or 2 wherein the target motif is a target sequence of nucleic acids.

4. The arrangement according to claim 3 wherein the second structure is a molecule having a single strand nucleic acid sequence binding specifically to a target sequence of nucleic acids whereby said single stranded nucleic acid sequence has a quencher moiety and a label moiety and said label is quenched when the quencher moiety is present in close vicinity at this structure and the target sequence is absent.

5. The arrangement according to any one of the preceding claims wherein the first structure is a nucleic acid sequence having attached thereto a plasmonic nanostructure, like metallic nanoparticle(s), optionally, whereby said nucleic acid sequences having attached thereto a plasmonic nanostructure, like the metallic nanoparticle(s), are positioned at predetermined segments of a two or three dimensional structure of predetermined shape whereby said two or three dimensional structure is formed of nucleic acid molecules, in particular, DNA molecules.

6. The arrangement according to any one of claims 1 to 4 whereby the metallic nanoparticles being bound to the two or three dimensional structure of predetermined shape whereby said structure is formed of nucleic acid molecules, in particular, DNA molecules, whereby said plasmonic nanostructure, like metallic nanoparticles, are arranged to allow formation of an area of electrical field enhancement due to plasmon resonance after excitation with electromagnetic waves.

7. The arrangement according to any one of the preceding claims wherein the two or three dimensional structure of predetermined shape is an array, preferably, comprising a multiple of nucleic acid molecules, in particular, a nucleic acid based origami structure.

8. The arrangement according to any one of the preceding claims wherein the second structure is a structure having a hairpin structure when no target sequence binds to the second structure, whereby the label and the quencher are present at the neck of the hairpin structure, thus, the label is quenched.

9. The arrangement according to any one of the preceding claims wherein the label is a chromophore, in particular, a fluorophore.

10. The arrangement according to any one of the preceding claims, whereby the metallic nanoparticles having a mean size of from 10 nm to 500 nm, preferably of from 10 nm to 200 nm.

11. The arrangement according to any one of the preceding claims, whereby the plasmonic nanostructure, like the metallic nanoparticles are selected from the plasmonic materials selected from silver, aluminum, gold, copper, palladium, and/or platinum.

12. The arrangement according to any one of the preceding claims, whereby the spacing of the at least two nanoparticles attached to the first structures and, optionally, said first structures are bound to an array including a DNA origami structure, is of from 2 nm to 50 nm.

13. A method for detecting and amplifying a signal based on a single target molecule containing a target motif comprising the steps of:
- providing an arrangement according to any one of claims 1 to 12;
- providing the target molecule containing the target motif;
- binding specifically the single target molecule to the second structure through binding of the target motif to the complementary target motif present in said second structure, thus, removing the quencher moiety from the vicinity of the label moiety,
- measuring the signal based on single target molecules by measuring the label excited by electromagnetic waves whereby said label is positioned in the area of electrical field enhancement due to plasmon resonance of the plasmonic nanostructure, like the metallic nanoparticle(s).

14. The method according to claim 13 wherein the label is a fluorophore and excitation is by laser light.

15. The use of an arrangement of any one of claims 1 to 12 or the method of claims 13 to 14 for i) signal enhancement, in particular, enhancement of fluorescence and/or ii) single molecule analysis and/or iii) in living cells.
